(19) Europäisches Patentamt
European Patent Office
Office européen des brevets

(11) EP 2 414 316 B1

(12) EUROPÄISCHE PATENTSCHRIFT

(45) Veröffentlichungstag und Bekanntmachung des Hinweises auf die Patenterteilung:
**30.11.2016 Patentblatt 2016/48**

(21) Anmeldenummer: **10711415.9**

(22) Anmeldetag: **30.03.2010**

(51) Int Cl.:
***C07C 68/02*** (2006.01) ***C07C 69/96*** (2006.01)
***C08F 212/08*** (2006.01) ***C08F 212/12*** (2006.01)
***C08F 220/06*** (2006.01) ***C08F 220/18*** (2006.01)
***C09D 133/08*** (2006.01) ***C09J 133/08*** (2006.01)

(86) Internationale Anmeldenummer:
**PCT/EP2010/054202**

(87) Internationale Veröffentlichungsnummer:
**WO 2010/112505 (07.10.2010 Gazette 2010/40)**

# (54) VERFAHREN ZUR HERSTELLUNG VON LÖSUNGEN VON STRAHLUNGSEMPFINDLICHEN, RADIKALISCH POLYMERISIERBAREN ORGANISCHEN VERBINDUNGEN

METHOD FOR PRODUCING SOLUTIONS OF RADIATION-SENSITIVE, RADICALLY POLYMERIZABLE ORGANIC COMPOUNDS

PROCÉDÉ DE PRÉPARATION DE SOLUTIONS DE COMPOSÉS ORGANIQUES POLYMÉRISABLES PAR VOIE RADICALAIRE, SENSIBLES AU RAYONNEMENT

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **02.04.2009 EP 09157168**

(43) Veröffentlichungstag der Anmeldung:
**08.02.2012 Patentblatt 2012/06**

(73) Patentinhaber: **BASF SE**
**67056 Ludwigshafen am Rhein (DE)**

(72) Erfinder:
- **WEISKOPF, Verena**
  **67583 Guntersblum (DE)**
- **WINDLIN, Franz Niklaus**
  **69120 Heidelberg (DE)**
- **WINSEL, Harald**
  **67251 Freinsheim (DE)**
- **RUCKPAUL, Markus**
  **69469 Weinheim (DE)**
- **WULFF, Dirk**
  **67105 Schifferstadt (DE)**
- **DÜSTERWALD, Uwe**
  **66851 Queidersbach (DE)**
- **LICHT, Ulrike**
  **68309 Mannheim (DE)**

(74) Vertreter: **BASF IP Association**
**BASF SE**
**ZRX-C6**
**67056 Ludwigshafen (DE)**

(56) Entgegenhaltungen:
**EP-A2- 0 377 191** **EP-A2- 1 213 306**
**WO-A1-03/070792** **JP-A- 2006 327 986**
**JP-B2- 4 038 600** **US-A- 2 587 437**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 414 316 B1

Printed by Jouve, 75001 PARIS (FR)

## Beschreibung

Die Erfindung betrifft ein Verfahren zur Herstellung von Lösungen von strahlungsempfindlichen, radikalisch polymerisierbaren organischen Verbindungen, wobei eine erste Ausgangsverbindung, welche eine Säurehalogenidgruppe aufweist, und eine zweite Ausgangsverbindung, welche eine alkoholische Hydroxygruppe aufweist, miteinander in einem Lösungsmittel oder in einem Lösungsmittelgemisch aus niedrig siedenden Ketonen verestert werden.

UV-vernetzbare Polyacrylate und ihre Verwendung für Schmelzhaftklebstoffe sind beschrieben in EP 377199 A, EP 655465 A und in EP 1213306 A. Die UV-vernetzbaren Polyacrylate enthalten einen durch radikalische Copolymerisation in die Polymerkette eingebauten Fotoinitiator. Geeignete copolymerisierbare Fotoinitiatormonomere sind in der EP 377191 A beschrieben. Als Verknüpfung zwischen photoreaktivem Molekülteil und polymerisierbarem Molekülteil enthalten die Fotoinitiatormonomere eine Estergruppe, insbesondere eine Carbonatestergruppe. Zur Herstellung wird ein geeignetes Säurehalogenid mit einer geeigneten Hydroxyverbindung verestert. Die Veresterung erfolgt in Gegenwart eines vorzugsweise im Überschuss eingesetzten Amins zum Abfangen der sich bildenden Säure unter Bildung eines Ammoniumhydrogenhalogenidsalzes. Amin und Ammoniumsalz müssen zuverlässig abgetrennt werden, da sie bei der nachfolgenden Copolymerisation und bei Verwendungen des Copolymerisats unerwünscht sind. So führen bereits sehr geringe Restmengen an Aminen zu Vergilbungen, was insbesondere die Anwendbarkeit als Klebstoff für durchsichtige Folien beeinträchtigt. Die Ammoniumsalze lassen sich am einfachsten mit Wasser auswaschen. Voraussetzung ist, dass sich zwischen dem Wasser und dem bei der Veresterungsreaktion eingesetzten organischen Lösungsmittel eine gute Phasentrennung ergibt. Sehr gut geeignet als Lösungsmittel sind aromatische Kohlenwasserstoffe wie z.B. Toluol oder Xylole, weil sie eine gute Phasentrennung mit Wasser ausbilden. Überschüssige aromatische Kohlenwasserstoffe können außerdem in einfacher Weise recycliert und für nachfolgende Synthesen wiederverwendet werden, da die recyclierten Lösungsmittel wasserfrei sind und eine Hydrolyse der Säurechloride nicht befürchtet werden muss. Ein weiterer Vorteil von höher siedenden Kohlenwasserstoffen wie z.B. Xylol liegt darin, dass überschüssige, niedrig siedende Amine wie z.B. Triethylamin in einfacher Weise destillativ abgetrennt werden können.

Die an sich sehr gut als Lösungsmittel bei der Herstellung der Fotoinitiatormonomeren geeigneten aromatischen Kohlenwasserstoffe haben allerdings den Nachteil, dass Restmengen in den nachfolgend hergestellten Copolymerisaten und den daraus hergestellten Endprodukten, z.B. in Schmelzhaftklebstoffen verbleiben und in nicht unerheblichem Maß zum Gesamtgehalt restflüchtiger Anteile (VOC-Gehalt) beitragen. Für viele endverbrauchernahe Anwendungen ist aber ein möglichst geringer VOC-Gehalt erwünscht. So kann beispielsweise bei Anwendungen als Klebeband im Innenbereich von Automobilen, z.B. als Bänder zur Umwickelung von Kabelbäumen (Kabelwickelbänder) oder als doppelseitige Klebebänder zur Befestigung von Zierteilen, ein zu hoher VOC-Gehalt des Klebstoffes zum sogenannten Fogging führen, der Bildung eines unerwünschten Niederschlags auf Glasscheiben. Für manche Anwendungen, z.B. in Pflastern mit direktem Hautkontakt ist zudem eine völlige Freiheit von aromatischen Kohlenwasserstoffen gewünscht.

Auch bei vielen Anwendungen von strahlungshärtbaren Beschichtungsmassen, z.B. bei der Härtung von Lack- und Harzüberzügen auf Papier, Metall oder Kunststoff oder bei der Trocknung von Druckfarben und Tinten ist ein möglichst geringer Gehalt an leichtflüchtigen Anteilen von großer Bedeutung. Derartige Anteile können die erzielbare Endhärte der Schicht beeinflussen oder es kann zu unerwünschten Farbveränderungen, z.B. Vergilbung kommen. Auch kann die Diffusion oder Migration aus der Beschichtung heraus in umgebende Materialien Probleme schaffen. Insbesondere wenn die Beschichtungen oder die diese umgebenden Materialien in Kontakt mit Lebensmitteln kommen ist ein möglichst geringer Gehalt bzw. die Freiheit von gesundheitlich bedenklichen Bestandteilen, z.B. aromatischen Kohlenwasserstoffen wünschenswert.

In der JP 4038600 B2 wird u.a. die Bildung einer Verbindung durch Reaktion von 1-(4-(2-Hydroxyethoxy)phenyl)-2-hydroxy-2-methyl-propan-1-on mit Methacryloyloxy-tetraethylenglycolchloroformiat in Methylethylketon beschrieben (siehe Beispiel 1).

Es bestand daher die Aufgabe, ein alternatives Verfahren für die Herstellung von Estergruppen enthaltenden, radikalisch polymerisierbaren Fotoinitiatormonomeren zur Verfügung zu stellen, bei welchem nach Möglichkeit einerseits auf die Verwendung von aromatischen Kohlenwasserstoffen als Lösungsmittel verzichtet werden kann und andererseits die Abtrennbarkeit von Aminen und Ammoniumsalzen ähnlich zuverlässig möglich ist wie bei der Verwendung von aromatischen Kohlenwasserstoffen und mit den Fotoinitiatormonomeren unmittelbar oder mittelbar hergestellte Endprodukte einen möglichst geringen VOC-Gehalt aufweisen.

Überraschenderweise wurde gefunden, dass die Aufgabe gelöst werden kann, indem als Lösungsmittel Ketone mit einem Siedepunkt von unter 150°C bei Normaldruck (1 bar) verwendet werden. Dies ist insofern überraschend, da diese Ketone in Unkenntnis der Erfindung aufgrund ihrer physikalischen Eigenschaften als für den beabsichtigten Zweck eher ungeeignet erscheinen. Die zum Teil mit Wasser mischbaren Ketone bilden mit Wasser häufig eine schlechtere Phasentrennung als Kohlenwasserstoffe, deshalb erscheint eine gute Abtrennung von organischer Phase und wässriger Phase fraglich. Organische Ammoniumsalze sind teilweise in Ketonen löslich, deshalb erscheint die Abtrennung aus der organischen Phase problematisch. Ketone sind zumindest teilweise mit Wasser mischbar. So lösen sich beispielsweise bis zu 12,5 Gew.% Wasser in Methylethylketon. Dies lässt eine Wiederverwendung von gebrauchtem Lösungsmittel schwierig erscheinen, da der Wassergehalt eine Hydrolyse der Säurechloride bewirken könnte. Außerdem sind Reste von überschüssigen Aminen nicht destillativ vom Lösungsmittel entfernbar, wenn der Siedepunkt des Lösungsmittels (z.B. 80°C für Methylethylketon) geringer ist als derjenige des abzutrennenden Amins (z.B. 89°C für Triethylamin). Die Schwierigkeiten, die Ketone als ungeeignet erscheinen lassen, konnten jedoch überwunden werden.

Gegenstand der Erfindung ist ein Verfahren zur Herstellung von Lösungen von strahlungsempfindlichen, radikalisch polymerisierbaren organischen Verbindungen, wobei

a) eine erste Ausgangsverbindung, welche eine Säurehalogenidgruppe aufweist, und
b) eine zweite Ausgangsverbindung, welche eine alkoholische Hydroxygruppe aufweist,

miteinander in einem Lösungsmittel oder in einem Lösungsmittelgemisch verestert werden; und
es sich bei dem Lösungsmittel um ein oder mehrere Ketone mit einem Siedepunkt von unter 150°C bei Normaldruck (1 bar) handelt oder das Lösungsmittelgemisch zu mindestens 50 Gew.%, bezogen auf die Lösungsmittelmenge, aus diesen Ketonen besteht; und
eine der beiden Ausgangsverbindungen mindestens eine strahlungsempfindliche Gruppe aufweist und die andere der beiden Ausgangsverbindungen mindestens eine ethylenisch ungesättigte, radikalisch polymerisierbare Gruppe aufweist, wobei es sich bei der strahlungsempfindlichen, radikalisch polymerisierbaren organischen Verbindung um einen polymerisierbaren Fotoinitiator handelt, welcher durch radikalische Copolymerisation in die Polymerkette von Copolymeren eingebaut werden kann,
wobei die Veresterung in Gegenwart von mindestens einem tertiären organischen Amin erfolgt, und
wobei im Anschluss an die Reaktion Wasser in einer Menge zugegeben wird, dass sich eine gesättigte Lösung des organischen Ammoniumhalogenids in Wasser bildet und diese gesättigte wässrige Lösung von der organischen Phase abgetrennt wird, wobei die Wassermenge so bemessen ist, dass sie die zur Bildung einer gesättigten Lösung benötigte Menge nicht um mehr als 10 Gew.% der theoretisch benötigten Menge überschreitet.

Im Folgenden wird gelegentlich die Bezeichnung (Meth)acrylat und ähnliche Bezeichnungen als abkürzende Schreibweise verwendet für "Acrylat oder Methacrylat". Strahlungsempfindliche, radikalisch polymerisierbare organische Verbindungen werden im Folgenden kurz als polymerisierbarer Fotoinitiator bezeichnet. Der polymerisierbare Fotoinitiator kann durch radikalische Copolymerisation in die Polymerkette von Copolymeren eingebaut werden. Polymerisierbare Fotoinitiatoren haben vorzugsweise folgenden prinzipiellen Aufbau:

$$A\text{-}X\text{-}B$$

wobei A ein einwertiger organischer Rest ist, welcher als strahlungsempfindliche Gruppe vorzugsweise eine Phenongruppe aufweist,

X eine Estergruppe ist, ausgewählt aus -O-C(=O)-, -(C=O)-O und -O-(C=O)-O-, und
B ein einwertiger organischer Rest ist, welcher eine ethylenisch ungesättigte, radikalisch polymerisierbare Gruppe enthält. Bevorzugte Reste A sind Reste, welche mindestens ein Strukturelement enthalten, welches abgeleitet ist von Phenonen, insbesondere von Acetophenonen oder Benzophenonen. Bevorzugte Reste B enthalten mindestens eine, vorzugsweise genau eine Acryl- oder Methacrylgruppe.

Die ethylenisch ungesättigte Gruppe kann direkt an die Gruppe X gebunden sein. Ebenso kann die strahlungsempfindliche Gruppe direkt an die Gruppe X gebunden sein. Es können sich aber auch zwischen ethylenisch ungesättigter Gruppe und der Gruppe X bzw. zwischen strahlungsempfindlicher Gruppe und Gruppe X jeweils eine Spacergruppe (Abstandshalter) befinden. Die Spacergruppe kann z.B. ein Molekulargewicht von bis zu 500, insbesondere bis zu 300 oder 200 g/Mol aufweisen.

Geeignete Fotoinitiatoren sind z.B. Verbindungen mit Acetophenon- oder Benzophenonstruktureinheiten, beispielsweise beschrieben in EP 377191 A oder EP 1213306 A. Eine bevorzugte Gruppe X ist die Carbonatgruppe -O-(C=O)-O-. Bevorzugte polymerisierbare Fotoinitiatoren sind Verbindungen der Formel:

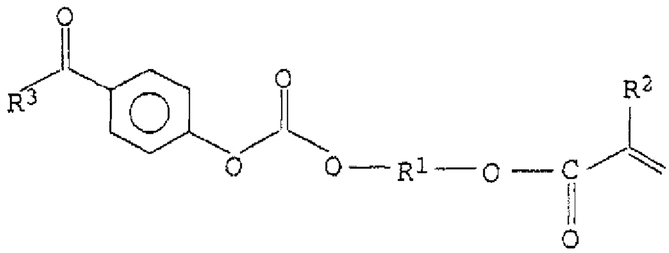

worin R1 für einen organischen Rest mit bis zu 30 C-Atomen, R2 für ein H-Atom oder eine Methylgruppe und R3 für eine substituierte oder unsubstituierte Phenylgruppe oder für eine C1-C4-Alkylgruppe steht. R1 steht besonders bevorzugt für eine Alkylengruppe, insbesondere für eine C2-C8-Alkylengruppe. R3 steht besonders bevorzugt für eine Methylgruppe oder für eine Phenylgruppe, ganz besonders bevorzugt für eine Phenylgruppe.

Weitere, als copolymerisierbare Fotoinitiatoren geeignete Acetophenon- und Benzophenonderivate sind z.B. solche der Formel

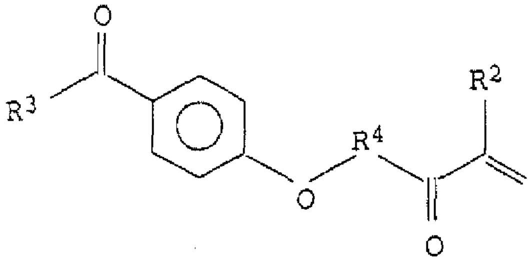

worin R2 und R3 die obige Bedeutung haben kann und R4 für eine Einfachbindung oder für (-CH2-CH2-O)n stehen kann, wobei n für eine ganze Zahl von 1 bis 12 steht.

Die erste Ausgangsverbindung zur Herstellung des polymerisierbaren Fotoinitiators weist eine Säurehalogenidgruppe auf. Bevorzugt ist eine Säurechloridgruppe, besonders bevorzugt eine Chlorformiatgruppe Cl-C(=O)-O-. Die zweite Ausgangsverbindung weist eine alkoholische Hydroxygruppe auf. Dabei kann es sich um eine aliphatische oder aromatische, insbesondere phenolische Hydroxygruppe handeln. Vorzugweise handelt es sich bei den copolymerisierbaren Fotoinitiatoren um Carbonate. Diese sind herstellbar durch Umsetzung von Kohlensäureesterchloriden mit Alkoholen. In einer bevorzugten Ausführungsform weist die erste Ausgangsverbindung eine Chlorformiatgruppe und eine Acrylat- oder Methacrylatgruppe auf und die zweite Ausgangsverbindung weist eine alkoholische Hydroxygruppe und eine Phenongruppe auf. Die Herstellung der Ausgangsverbindungen ist nach bekannten Verfahren möglich. eine Zusammenstellung findet sich beispielsweise in der EP 377 191 A.

Erfindungsgemäß erfolgt die Herstellung des polymerisierbaren Fotoinitiators durch Veresterung in einem Lösungsmittel oder in einem Lösungsmittelgemisch. Bei dem Lösungsmittel oder dem Lösungsmittelgemisch handelt es sich um ein oder mehrere Ketone mit einem Siedepunkt von unter 150°C, vorzugsweise unter 130°C bei Normaldruck (1 bar) bzw. das Lösungsmittelgemisch besteht zu mindestens 50 Gew.%, bezogen auf die Lösungsmittelmenge, aus diesen Ketonen. Der Siedebereich der Lösungsmittel ist vorzugsweise von 50 bis unter 150 °C, insbesondere von 60 bis 120 °C. Bevorzugt sind Dialkylketone mit einem Siedepunkt im Bereich von 50 bis unter 130 °C. Der Gehalt an polymerisierbarem Fotoinitiator in den erfindungsgemäß hergestellten Lösungen beträgt vorzugsweise von 5 bis 85 Gew.%, vorzugsweise 20 bis 60 Gew.-%. Geeignete Ketone haben im Allgemeinen ein Molgewicht unter 150g/mol. Genannt seien z.B. Aceton, Methylethylketon (MEK, 2-Butanon) oder Methylisobutylketon (MIBK). Besonders bevorzugt sind MEK und MIBK. Die Ketone oder Gemische dieser Ketone sind besonders bevorzugt als alleiniges Lösungsmittel. Sie können aber auch im Gemisch mit anderen Lösungsmitteln vorliegen. Im Falle eines derartigen Lösungsmittelgemischs beträgt der Anteil der Ketone mindestens 50 Gew.-% bezogen auf die gesamte Lösungsmittelmenge, bevorzugt beträgt er mindestens 80 Gew.-%, besonders bevorzugt mindestens 95 Gew.-%. Ganz besonders bevorzugt handelt es sich bei dem Lösungsmittel allein und ausschließlich um Methylethylketon oder um Methylisobutylketon.

Überraschenderweise wurde gefunden, dass trotz der Hydrolyseempfindlichkeit des Säurehalogenids nicht unbedingt unter völligem Feuchtigkeitsausschluss gearbeitet werden muss. Dies ermöglicht den Einsatz insbesondere von Lösungsmitteln oder Lösungsmittelgemischen, die nicht völlig wasserfrei sind. Hierdurch ist es möglich, dass zumindest ein Teil der als Lösungsmittel eingesetzten Ketone recyclierte, wasserhaltige Ketone sind, welche aus einem vorhergehenden Herstellungsverfahren z.B. durch azeotrope Destillation zurückgewonnen wurden. So kann beispielsweise Methylethylketon bis zur Sättigungsgrenze (ca. 12,5 Gew.%) Wasser, z.B. mindestens 1 oder mindestens 5

Gew.% Wasser enthalten.

Die Ausgangsverbindungen werden vorzugsweise im äquimolaren Verhältnis eingesetzt, optional kann aber auch ein Überschuss von bis zu 10 bis 30 mol% einer der Ausgangsverbindungen eingesetzt werden. Die Veresterung der Ausgangsverbindungen erfolgt vorzugsweise in Gegenwart eines basischen, nicht nucleophilen tertiären Amins. In der Regel wird eine Lösung oder Suspension der Hydroxyverbindung in dem Lösungsmittel in Gegenwart eines basischen, nicht nucleophilen Amins vorgelegt und das Säurehalogenid wird zugegeben. Amine sind z.B. Triethylamin, 4-Dimethylaminopyridin, Imidazol, 1,4-Diazabicyclo[2.2.2]octan, 1,5-Diazabicyclo[4.3.0]non-5-en, 1,8-Diazabicyclo[5.4.0]undec-7-en, Polyvinylpyridin, N,N'-Dimethylpropylenharnstoff oder N,N'-Dimethylethylenharnstoff. Besonders bevorzugt sind tertiäre organische Amine der Formel $NR_3$, wobei R eine Alkylgruppe mit 1 bis 10, vorzugsweise 2 bis 4 C-Atomen ist.

Die Menge an eingesetztem Amin ist vorzugsweise im Wesentlichen stöchiometrisch, d.h. die Menge an Amin ist äquimolar zur Menge an Säurehalogenid, optional kann aber auch ein Überschuss von bis zu 5 oder bis zu 10 mol% an Amin eingesetzt werden. Die Säurehalogenidverbindung wird (optional gelöst in dem Lösungsmittel des Reaktionsmediums) unter Rühren zugetropft. Die Veresterungsreaktion erfolgt vorzugsweise bei Temperaturen von 0 bis 100 °C, vorzugsweise bei 10 bis 50°C. Nach einer Nachrührzeit von 1 bis 48 Stunden, vorzugsweise 1 bis 20 Stunden bei z.B. 10 bis 50 °C werden die gebildeten Ammoniumsalze und gegebenenfalls überschüssiges Amin nach Zugabe von Wasser oder verdünnter Salzsäure mittels Phasentrennung abgetrennt. Überschüssiges Amin kann weiterhin destillativ entfernt werden.

Um eine vorzeitige Polymerisation der radikalisch polymerisierbaren Edukte und Produkte zu verhindern, setzt man dem Reaktionsgemisch vorzugsweise einen üblichen Stabilisator zu. Als solche kommen z.B. Hydrochinon, Hydrochinonmonomethylether, 2,6-Di-tert.-butyl-4-methylphenol, para-Nitrosophenol, 2,2,6,6-Tetramethylpiperidinyloxyl (TEMPO) und/oder Phenothiazin in Betracht. Darüber hinaus hat es sich als äußerst vorteilhaft erwiesen, während der Acylierung Sauerstoff oder Luft mit einem Sauerstoffgehalt von 1 - 20% durch das Reaktionsgemisch zu leiten.

Überraschenderweise hat sich gezeigt, dass sich trotz der schlechten Phasentrennung von reinem Ketonlösungsmittel und Wasser, der gegenseitigen teilweisen Mischbarkeit von Keton und Wasser sowie trotz der teilweisen Löslichkeit von organischen Ammoniumverbindungen in Ketonen, die sich bei der Veresterungsreaktion bildenden organischen Ammoniumverbindungen hinreichend gut von der Lösung des Fotoinitiators in Keton abtrennen lassen. Als besonders zweckmäßig hat es sich erwiesen, die Abtrennung des sich bei der Reaktion bildenden organischen Ammoniumhalogenids in der Weise durchzuführen, dass im Anschluss an die Reaktion Wasser in einer Menge zugegeben wird, dass sich eine im wesentlichen gesättigte Lösung des organischen Ammoniumhalogenids in Wasser bildet und diese gesättigte wässrige Lösung von der organischen Phase abgetrennt wird. Die Wassermenge ist so bemessen, dass sie die zur Bildung einer gesättigten Lösung benötigte Menge einerseits nicht überschreitet oder nicht wesentlich, d.h. nicht um mehr als 10 Gew.%, vorzugsweise nicht um mehr als 5 Gew.% der theoretisch benötigten Menge überschreitet. Andererseits soll die Wassermenge möglichst ausreichend sein, damit die Ammoniumhalogenide vollständig oder fast vollständig, d.h. zu mindestens 90 Gew.%, vorzugsweise zu mindestens 95 Gew.% gelöst sind. Die gesättigte wässrige Ammoniumsalzlösung bildet dabei eine gute Phasentrennung mit der organischen Phase und ist gut abtrennbar.

In der organischen Phase gelöst verbliebene, geringe Anteile an Ammoniumhalogeniden können durch Tieftemperaturausfällung abgetrennt werden. Hierzu wird die Temperatur der organischen Phase soweit unterhalb Raumtemperatur abgesenkt, dass die Ammoniumhalogenide aus der organischen Phase auskristallisieren. Vorzugsweise wird auf 0°C oder darunter abgekühlt, besonders bevorzugt auf -5°C oder darunter oder auf -10°C, oder darunter. Die ausgefallenen Salze können abfiltriert werden.

Vorzugsweise werden Amine nur in stöchiometrischen Mengen eingesetzt. Falls dennoch nach der Reaktion überschüssige Restmengen an Aminen vorhanden sind, können diese durch Neutralisation mit Säure in Ammoniumsalze überführt und wie oben abgetrennt werden und/oder restliche Amine können destillativ, z.B. als Azeotrop entfernt werden. Insbesondere durch die Kombination der verschiedenen Maßnahmen wie (i) weitestgehende Vermeidung von überschüssigen Aminen durch Verwendung stöchiometrischer Mengen, (ii) Überführung in Ammoniumsalze und dessen Abtrennung sowie (iii) destillative Reinigung ist es möglich, die Amine soweit zu entfernen, dass eine durch Aminspuren verursachte Vergilbung von Endprodukten, zu dessen Herstellung der copolymerisierbare Fotoinitiator eingesetzt wird, vermieden werden kann.

Die oben beschriebenen Lösungen copolymerisierbarer Fotoinitiatoren können zur Herstellung von strahlungshärtbaren Materialien, insbesondere auf Basis von strahlungsvernetzbaren, radikalisch copolymerisierten Copolymeren verwendet werden. Verwendungen sind z.B. die Herstellung von strahlungshärtbaren Schmelzhaftklebstoffen, strahlungshärtbaren wässrigen Polymerdispersionen, strahlungshärtbaren Beschichtungsmaterialien, strahlungshärtbaren Lacken, strahlungshärtbaren Druckfarben, strahlungshärtbaren Tinten, strahlungshärtbaren Siebdruckmaterialien und strahlungshärtbaren Oberflächenbeschichtungen von Lebensmittelverpackungen.

Besonders bevorzugt ist die Verwendung zur Herstellung von Schmelzhaftklebstoffen oder von wässrigen Polymerdispersionen, z.B. für die Herstellung von UV-härtbaren Klebstoffen oder für die Herstellung von UV-härtbaren Beschichtungsmitteln. Vorzugsweise handelt es sich bei den Copolymeren um Polyacrylatcopolymere. Dabei handelt es sich um ein Polymer, welches durch radikalische Polymerisation von Acrylmonomeren, worunter auch Methacrylmonomere verstanden werden, und weiteren, copolymerisierbaren Monomeren erhältlich ist. Bevorzugt ist das Polyacrylatcopolymer zu mindestens 40 Gew.-% besonders bevorzugt zu mindestens 60 Gew.-%, ganz besonders bevorzugt zu mindestens 80 Gew.-% aus C1 - C10 Alkyl(meth)acrylaten aufgebaut. Genannt seien insbesondere C1-C8 Alkyl(meth)acrylate, z.B. Methyl(meth)-acrylat, Ethyl(meth)acrylat, n-Butyl(meth)acrylat, 2-Ethylhexyl (meth)acrylat. Es handelt sich um ein mit UV-Licht vernetzbares Polyacrylatcopolymer. Der Fotoinitiator ist an das Polyacrylat gebunden. Durch Bestrahlung mit energiereichem Licht, insbesondere UV-Licht bewirkt der Fotoinitiator eine Vernetzung des Polymeren, vorzugsweise durch eine chemische Pfropfreaktion des Fotoinitiators mit einer räumlich benachbarten Polymerkette. Insbesondere kann die Vernetzung durch Einschub einer Carbonylgruppe des Fotoinitiators in eine benachbarte C-H-Bindung unter Ausbildung einer -C-C-O-H Gruppierung erfolgen. Das Polyacrylatcopolymer enthält vorzugsweise 0,0001 bis 1 mol, besonders bevorzugt 0,0002 bis 0,1, ganz besonders bevorzugt 0,0003 bis 0,01 mol des Fotoinitiators, bzw. der als Fotoinitiator wirksamen, an das Polymer gebundenen Molekülgruppe, pro 100 g Polyacrylatcopolymer.

Weitere, von Acrylaten verschiedene Monomere, aus denen das Polyacrylatcopolymer zusätzlich aufgebaut sein kann, sind z.B. Vinylester von bis zu 20 C-Atome enthaltenden Carbonsäuren, Vinylaromaten mit bis zu 20 C-Atome, ethylenisch ungesättigten Nitrile, Vinylhalogenide, Vinylether von 1 bis 10 C-Atome enthaltenden Alkoholen, aliphatischen Kohlenwasserstoffe mit 2 bis 8 C-Atomen und 1 oder 2 Doppelbindungen oder Mischungen dieser Monomeren. Als vinylaromatische Verbindungen kommen z.B. Vinyltoluol a- und p-Methylstyrol, alpha-Butylstyrol, 4-n-Butylstyrol, 4-n-Decylstyrol und vorzugsweise Styrol in Betracht. Beispiele für Nitrile sind Acrylnitril und Methacrylnitril. Die Vinylhalogenide sind mit Chlor, Fluor oder Brom substituierte ethylenisch ungesättigte Verbindungen, bevorzugt Vinylchlorid und Vinylidenchlorid. Als Vinylether zu nennen sind z. B. Vinylmethylether oder Vinylisobutylether. Bevorzugt sind Vinylether von 1 bis 4 C-Atome enthaltenden Alkoholen. Als Kohlenwasserstoffe mit 2 bis 8 C-Atomen und zwei olefinischen Doppelbindungen seien Butadien, Isopren und Chloropren genannt. Als weitere Monomere in Betracht kommen insbesondere auch Monomere mit Carbonsäure-, Sulfonsäure- oder Phosphonsäuregruppen. Bevorzugt sind Carbonsäuregruppen. Genannt seien z. B. Acrylsäure, Methacrylsäure, Itaconsäure, Maleinsäure oder Fumarsäure. Weitere Monomere sind z. B. auch (Meth)acrylamid und Hydroxylgruppen enthaltende Monomere, insbesondere C1-C10-Hydroxyalkyl(meth)acrylate. Darüber hinaus seien Phenyloxyethylglykolmono-(meth)acrylat, Glydidylacrylat, Glycidylmethacrylat, Amino-(meth)acrylate wie 2-Aminoethyl(meth)acrylat genannt. Monomere, die außer der Doppelbindung noch weitere funktionelle Gruppen tragen, z. B. Isocyanat-, Amino-, Hydroxy-, Amid- oder Glycidyl-, können z. B. die Haftung auf Substraten verbessern.

Das Polyacrylatcopolymer hat vorzugsweise einen K-Wert von 30 bis 80, besonders bevorzugt von 40 bis 60, gemessen in Tetrahydrofuran (1%ige Lösung, 21 °C). Der K-Wert nach Fikentscher ist ein Maß für das Molekulargewicht und die Viskosität des Polymerisats. Die Glasübertragungstemperatur (Tg) des Polyacrylatcopolymeren beträgt vorzugsweise -60 bis +10 °C, besonders bevorzugt -55 bis 0 °C, ganz besonders bevorzugt -55 bis -10 °C. Die Glasübertragungstemperatur lässt sich nach üblichen Methoden wie Differentialthermoanalyse oder Differential Scanning Calorimetrie (s. z.B. ASTM 3418/82, sog. "midpoint temperature") bestimmen.

Die Polyacrylatcopolymere können durch Copolymerisation der monomeren Komponenten unter Verwendung der üblichen Polymerisationsinitiatoren sowie gegebenenfalls von Reglern hergestellt werden, wobei man bei den üblichen Temperaturen in Substanz, in Emulsion, z.B. in Wasser oder flüssigen Kohlenwasserstoffen, oder in Lösung polymerisiert. Vorzugsweise werden die Polyacrylatcopolymere entweder durch Emulsionspolymerisation in Wasser oder durch Polymerisation der Monomeren in organischen Lösungsmitteln, insbesondere in organischen Lösungsmitteln eines Siedebereichs von 50 bis 150 °C, vorzugsweise von 60 bis 120 °C unter Verwendung der üblichen Mengen an Polymerisationsinitiatoren, die im allgemeinen bei 0,01 bis 10, insbesondere bei 0,1 bis 4 Gew.-%, bezogen auf das Gesamtgewicht der Monomeren liegt, hergestellt.

Die Copolymerisate können bei Temperaturen von 20 bis 150 °C, vorzugsweise bei Temperaturen im Bereich von 70 bis 120 °C und Drucken von 0,1 bis 100 bar (absolut), bevorzugt bei 0,3 bis 10 bar, in Gegenwart von 0,01 bis 10 Gew.-% an Peroxiden oder Azostartern als Polymerisationsinitiatoren, bezogen auf die Monomeren und in Gegenwart von 0 bis 200 Gew.-% an indifferenten Lösungsmitteln, bevorzugt 5 bis 25 Gew.-%, bezogen auf die Monomeren, d.h. durch Lösungs- oder Substanzpolymerisation hergestellt werden. Vorzugsweise erfolgt die Reaktion unter zunehmendem Vakuum, z.B. durch Absenkung des Drucks von Normaldruck (1 bar) auf 500 mbar (absolut). Lösungsmittel sind beispielsweise Kohlenwasserstoffe, Alkohole wie Methanol, Ethanol, Propanol, Butanol, Isobutanol, Ketone wie Aceton, Methylethylketon, Methylisobutylketon, Essigsäureethylester, Nitrile wie Acetonitril und Benzonitril oder Gemische aus den genannten Lösemitteln.

In einer bevorzugten Ausführungsform wird als Lösungsmittel für die Polymerisation das gleiche Lösungsmittel verwendet wie für die erfindungsgemäße Herstellung des copolymerisierbaren Photoinitiators, d.h. ein oder mehrere Ketone mit einem Siedepunkt von unter 150°C bei Normaldruck (1 bar).

Als Polymerisationsinitiatoren kommen beispielsweise Azoverbindungen, Ketonperoxide und Alkylperoxide in Betracht, z.B. Acylperoxide wie Benzoylperoxid, Dilauroylperoxid, Didecanoylperoxid, Isononanoylperoxid, Alkylester wie tert.-Butyl-tert.-pivalat, tert.-Butyl-per-2-ethylhexanoat, tert.-Butyl-per-maleinat, tert.-Butyl-per-isononanoat, tert.-Butyl-per-benzoat, tert.-Amylper-2-ethylhexanoat, Dialirylperoxide wie Dicumylperoxid, tert.-Butylcumylperoxid, Di-tert.-Butyl-peroxid und Peroxodicarbonate. Des Weiteren können als Initiatoren Azostarter wie beispielsweise 2,2'-Azobisisobutyronitril, 2,2'-Azobis(methylisobutyrat) oder 2,2'-Azobis(2,4-dimethylvaleronitril) Verwendung finden.

Für die Durchführung der Polymerisation können dem Reaktionsgemisch auch den Polymerisationsgrad senkende Verbindungen, sogenannte Polymerisationsregler zugesetzt werden, z.B. in Mengen von 0,1 bis 0,8 Gew.-Teile, bezogen auf 100 Gew.-Teile der zu polymerisierenden Monomeren. Geeignet sind z.B. Verbindungen mit einer Thiolgruppe beispielsweise Mercaptane wie Mercaptoethanol, tert.-Butylmercaptan, Mercaptobernsteinsäure, Thioglycolsäureethylacrylester, 3-Mercaptopropyltrimethoxysilan oder Dodecylmercaptan. In einer Ausführungsform werden keine Molekulargewichtsregler eingesetzt.

Die Lösung des copolymerisierbaren Fotoinitiators ist auch verwendbar in einem Verfahren zur Herstellung von wässrigen Polymerdispersionen, wobei

(i) eine oben beschriebene Lösung eines copolymerisierbaren Fotoinitiators zur Verfügung gestellt wird und
(ii) die in der Lösung enthaltene strahlungsempfindliche, radikalisch polymerisierbare organische Verbindung mit davon verschiedenen, radikalisch polymerisierbaren Monomeren unter Bildung von strahlungsvernetzbaren Copolymeren durch Emulsions- oder Suspensionspolymerisation in Gegenwart einer wässrigen Phase radikalisch copolymerisiert wird.

Die Herstellung der Polymere erfolgt vorzugsweise als Emulsionspolymerisat durch Emulsionspolymerisation, Die Emulsionspolymerisation erfolgt unter Verwendung von Emulgatoren und/oder Schutzkolloiden bzw. Stabilisatoren als grenzflächenaktive Substanzen. Vorzugsweise werden als grenzflächenaktive Substanzen ausschließlich Emulgatoren eingesetzt, deren Molekulargewicht im Unterschied zu den Schutzkolloiden üblicherweise unter 2000 g/mol liegen. Vorzugsweise werden anionische und nichtionische Emulgatoren als grenzflächenaktive Substanzen verwendet. Gebräuchliche Emulgatoren sind z.B. ethoxylierte Fettalkohole (EO-Grad: 3 bis 50, Alkylrest: $C_8$-bis $C_{36}$), ethoxylierte Mono-, Di- und Trialkylphenole (EO-Grad: 3 bis 50, Alkylrest: $C_4$-bis $C_9$), sowie Alkali- und Ammoniumsalze von Alkylsulfaten (Alkylrest: $C_8$- bis $C_{12}$), von ethoxylierten Alkanolen (EO-Grad: 4 bis 30, Alkylrest: $C_{12}$- bis $C_{18}$), von ethoxylierten Alkylphenolen (EO-Grad: 3 bis 50, Alkylrest: $C_4$- bis $C_9$), von Alkylsulfonsäuren (Alkyl-rest: $C_{12}$- bis $C_{18}$) und von Alkylarylsulfonsäuren (Alkylrest: $C_9$- bis $C_{18}$). Handelsprodukte geeigneter Emulgatoren sind z.B. Dowfax®2 A1, Emulan® NP 50, Dextrol® OC 50, Emulgator 825, Emulgator 825 S, Emulan®OG, Texapon® NSO, Nekanil® 904 S, Disponil® FES 77, Lutensol® AT 18, Steinapol VSL, Emulphor NPS 25.

Die Emulsionspolymerisation kann mit wasserlöslichen Initiatoren gestartet werden. In einer bevorzugten Ausführungsform werden eine oder mehrere Initiatoren eingesetzt, wobei die Gesamtmenge an Initiator weniger als 0,4 pphm (parts per hundred monomer; Gewichtsteile pro hundert Gewichtsteile Monomer), insbesondere maximal 0,3 pphm, z.B. von 0,1 oder von 0,2 bis 0,3 pphm beträgt. Wasserlösliche Initiatoren sind z.B. Ammonium- und Alkalimetallsalze der Peroxodischwefelsäure, z.B. Natriumperoxodisulfat, Wasserstoffperoxid oder organische Peroxide, z.B. tert-Butylhydroperoxid. Als Initiator geeignet sind auch so genannte Reduktions-Oxidations(Red-Ox)-Initiator Systeme. Die Red-Ox-Initiator-Systeme bestehen aus mindestens einem meist anorganischen Reduktionsmittel und einem anorganischen oder organischen Oxidations-mittel. Bei der Oxidationskomponente handelt es sich z.B. um die bereits vorstehend genannten Initiatoren für die Emulsionspolymerisation. Bei der Reduktionskomponente handelt es sich z.B. um Alkalimetallsalze der schwefligen Säure, wie z.B. Natriumsulfit, Natriumhydrogensulfit, Alkalisalze der dischwefligen Säure wie Natriumdisulfit, Bisulfitadditionsverbindungen aliphatischer Aldehyde und Ketone, wie Acetonbisulfit oder Reduktionsmittel wie Hydroxymethansulfinsäure und deren Salze, oder Ascorbinsäure. Die Red-Ox-Initiator-Systeme können unter Mitverwendung löslicher Metallverbindungen, deren metallische Komponente in mehreren Wertigkeitsstufen auftreten kann, verwendet werden. Übliche Red-Ox-Initiator-Systeme sind z.B. Ascorbinsäure/Eisen(II)sulfat/Natriumperoxidisulfat, tert-Butylhydroperoxid/Natriumdisulfit, tert-Butylhydroperoxid/Na-Hydroxymethansulfinsäure. Die einzelnen Komponenten, z.B. die Reduktionskomponente, können auch Mischungen sein z.B. eine Mischung aus dem Natriumsalz der Hydroxymethansulfinsäure und Natriumdisulfit. Es können auch mehrere, verschiedene Initiatoren bei der Emulsionspolymerisation Verwendung finden.

Das Polymerisationsmedium kann sowohl nur aus Wasser, als auch aus Mischungen aus Wasser und damit mischbaren Flüssigkeiten wie Methanol bestehen. Vorzugsweise wird nur Wasser verwendet. Die Emulsionspolymerisation kann sowohl als Batchprozess als auch in Form eines Zulaufverfahrens, einschließlich Stufen- oder Gradientenfahrweise, durchgeführt werden. Bevorzugt ist das Zulaufverfahren, bei dem man einen Teil des Polymerisationsansatzes vorlegt, auf die Polymerisationstemperatur erhitzt, anpolymerisiert und anschließend den Rest des Polymerisationsansatzes kontinuierlich, stufenweise oder unter Überlagerung eines Konzentrationsgefälles unter Aufrechterhaltung der Polymerisation der Polymerisationszone zuführt. Bei der Polymerisation kann auch z.B. zur besseren Einstellung der Teilchengröße eine Polymersaat vorgelegt werden.

Bei der Emulsionspolymerisation werden wässrige Dispersionen des Polymeren in der Regel mit Feststoffgehalten von 15 bis 75 Gew.-%, bevorzugt von 20 bis 70 Gew.% oder von 40 bis 70 Gew.-% erhalten. In einer Ausführungsform enthält die Dispersion bzw. der Haftklebstoff mindestens 60 Gew.% dispergiertes Polymer. Um Feststoffgehalte > 60 Gew.-% erreichen zu können, sollte man eine bi- oder polymodale Teilchengröße einstellen, da sonst die Viskosität zu hoch wird und die Dispersion nicht mehr handhabbar ist. Die Erzeugung einer neuen Teilchengeneration kann beispielsweise durch Zusatz von Saat vor oder während der Emulsionspolymerisation, durch Zugabe überschüssiger Emulgatormengen oder durch Zugabe von Miniemulsionen erfolgen. Ein weiterer Vorteil, der mit der niedrigen Viskosität bei hohem Feststoffgehalt einhergeht, ist das verbesserte Beschichtungsverhalten bei hohen Feststoffgehalten. Die Erzeugung einer oder mehrerer neuer Teilchengenerationen kann zu einem beliebigen Zeitpunkt erfolgten. Er richtet sich nach der für eine niedrige Viskosität angestrebten Teilchengrößenverteilung.

Die durch Emulsionspolymerisation hergestellten Copolymere werden vorzugsweise in Form einer wässrigen Dispersion verwendet. Die mittlere Teilchengröße der in der wässrigen Dispersion dispergierten Polymerteilchen beträgt vorzugsweise von 100 bis 500 nm. Die Größenverteilung der Dispersionsteilchen kann monomodal, bimodal oder multimodal sein. Bei monomodaler Teilchengrößenverteilung ist die mittlere Teilchengröße der in der wässrigen Dispersion dispergierten Polymerteilchen vorzugsweise kleiner 500 nm. Bei bi- oder multimodaler Teilchengrößenverteilung kann die Teilchengröße auch bis zu 1000 nm betragen. Unter mittlerer Teilchengröße wird hier der $d_{50}$-Wert der Teilchengrößenverteilung verstanden, d.h. 50 Gew.-% der Gesamtmasse aller Teilchen haben einen kleineren Teilchendurchmesser als der $d_{50}$-Wert. Die Teilchengrößenverteilung kann in bekannter Weise mit der analytischen Ultrazentrifuge (W. Mächtle, Makromolekulare Chemie 185 (1984), Seite 1025 - 1039) bestimmt werden.

Ein Vorteil der durch Emulsionspolymerisation hergestellten Polymerdispersionen besteht darin, dass es in einfacher Weise möglich ist, UV-vernetzbare Dispersionen zur Verfügung zu stellen, die frei von restflüchtigen aromatischen Kohlenwasserstoffen sind und die insgesamt einen sehr geringen Anteil flüchtiger organischer Verbindungen (VOC) aufweisen. Reste des bei der Herstellung des Fotoinitiatormonomers verwendeten Ketonlösungsmittels können in einfacher Weise durch azeotrope Destillation aus der wässrigen Dispersion entfernt werden.

Die Lösung des copolymerisierbaren Fotoinitiators ist auch verwendbar in einem Verfahren zur Herstellung von Schmelzhaftklebstoffen, wobei

(i) eine oben beschriebene Lösung eines copolymerisierbaren Fotoinitiators zur Verfügung gestellt wird und
(ii) die in der Lösung enthaltene strahlungsempfindliche, radikalisch polymerisierbare organische Verbindung mit davon verschiedenen, radikalisch polymerisierbaren Monomeren unter Bildung von strahlungsvernetzbaren Copolymeren, vorzugsweise strahlungsvernetzbaren Polyacrylaten, in einem organischen Lösungsmittel radikalisch copolymerisiert wird, und
(iii) das organische Lösungsmittel entfernt wird.

Es besteht ein Bedarf, die restflüchtigen Anteile, insbesondere nicht umgesetzte Monomere und Lösemittelreste auf ein Minimum abzusenken. Für die Lösemittelabtrennung (Abdampfen des Lösemittels unter Zurückbehaltung der Polyacrylatschmelze) bieten sich sehr verschiedene technische Verfahren an, z.B. die Kesseldestillation, die Verwendung eines Fallfilmverdampfers, die Strangentgasung oder die Restentgasung im Extruder. Grundsätzlich existiert eine Vielzahl von Verfahren zur Abtrennung restflüchtiger Anteile aus Polymer-Schmelzen. Eine wirtschaftliche Entgasung von höherviskosen Kunststoff-Schmelzen geschieht beispielsweise durch Strangentgasen oder Behandlung der Produkte im Entgasungsextruder. Eine bevorzugte Methode ist das in der EP 655465 A beschriebene Verfahren zur Beseitigung restflüchtiger Anteile aus Polyacrylatschmelzen. Hierbei erfolgt das Abdampfen der flüchtigen Anteile im Vakuum und ein Schleppmittel wie Wasserdampf, Stickstoff, Argon, CO2 wird gegen Ende der Destillation unter Vakuumbedingungen in die heiße Polyacrylatschmelze (z.B. bei Temperaturen von über 100 °C) gepresst, wobei die Schmelzen gleichzeitig umgepumpt werden. Anschließend wird das Schleppmittel gemeinsam mit den restflüchtigen Anteilen abgezogen. Besonders vorteilhaft ist Wasserdampf als Schleppmittel.

Der Schmelzhaftklebstoff eignet sich zur Herstellung von Schmelzhaftklebstoffbeschichtungen, z.B. auf Etiketten, Klebebändern und Folien. Die Etiketten können z.B. aus Papier oder Kunststoffen wie Polyester, Polyolefine oder PVC sein. Die Klebebänder oder Folien können ebenfalls aus den vorstehenden Kunststoffen sein. Zur Herstellung der Beschichtungen können die Schmelzhaftklebstoffe vorzugsweise als Schmelze auf die zu beschichtenden Substrate aufgetragen werden, d.h. das Lösungsmittel wird vorher nach geeigneten Verfahren entfernt, vorzugsweise auf einen Restgehalt von kleiner 0,5 Gew.%, bezogen auf das Polyacrylatcopolymer. Danach kann die Zusammensetzung als Schmelze, d.h. im Allgemeinen bei Temperaturen von 80 bis 160 °C auf Substrate, wie sie z.B. oben genannt sind, aufgetragen werden. Bevorzugte Schichtdicken sind z.B. 1 bis 200 Mikrometer, besonders bevorzugt 2 bis 80, ganz besonders bevorzugt 5 bis 80 Mikrometer. Die mit UV-Licht vernetzbaren Polyacrylatcopolymere können dann mit energiereicher Strahlung, vorzugsweise UV-Licht bestrahlt werden, so dass eine Vernetzung erfolgt. Im Allgemeinen werden die beschichteten Substrate dazu auf ein Transportband gelegt und das Transportband an einer Strahlungsquelle, z.B. einer UV-Lampe vorbeigeführt. Der Vernetzungsgrad der Polymerisate hängt von der Dauer und der Intensität der Bestrahlung ab. Vorzugsweise beträgt die Strahlungsenergie insgesamt 100 bis 1500 mJ/cm$^2$ bestrahlte Fläche. Die erhaltenen, beschichteten Substrate können vorzugsweise als Selbstklebeartikel, wie Etiketten, Klebebänder oder

Schutzfolien Verwendung finden. Die erhaltenen, mit UV-Licht vernetzten Schmelzklebstoffbeschichtungen haben gute anwendungstechnische Eigenschaften, z.B. eine gute Haftung und hohe innere Festigkeit und zeichnen sich durch besonders geringe Anteile an flüchtigen organischen Stoffen (VOC), insbesondere Freiheit von aromatischen Kohlenwasserstoffen aus.

Die Copolymerisate können außerdem als UV-vernetzbare Massen zur Herstellung von Überzügen, Beschichtungen und Imprägnierungen, insbesondere zur Herstellung von Haftklebstoffen, Haftklebefolien, Haftklebebändern, Haftklebeetiketten sowie Prägezangenfolien verwendet werden. Dabei können die Massen in an sich üblicher Weise durch Streichen, Spritzen, Walzen, Rakeln oder Giessen, gegebenenfalls bei erhöhter Temperatur, meist im Temperaturbereich von 20 bis 180 °C, auf übliche Substrate aufgebracht werden, beispielsweise auf Papier, Pappe, Holz, Glas, Metalle, Metall- und Kunststoffolien, beispielsweise auf weichgemachtes PVC, Polypropylen, Polyethylen, Polyamiden, Polyestern oder Aluminium und Kupfer. Weiterhin lassen sich auch Vliesstoffe, Fasern, Leder und textile Gewebe beschichten. Die Copolymerisate können auch beispielsweise zur Herstellung von Haftklebeetiketten im Transferauftrag auf Träger wie Papier aufgebracht werden, indem sie zunächst auf abhäsiv beschichteten Trägermaterialien, beispielsweise silikonisiertem Papier aufgebracht und im Falle der UV-vernetzbaren Massen bestrahlt und anschließend beispielsweise auf Papier kaschiert werden. Nach dem Abziehen des silikonisierten Papiers kann die haftklebrige Schicht gegebenenfalls nochmals bestrahlt werden. Als UV-Strahler können die üblichen Strahler, beispielsweise Quecksilbermitteldrucklampen mit einer Strahlungsleistung von 80 bis 240 Watt/cm eingesetzt werden. Die Haftklebemittel können in an sich üblicher Form modifiziert und/oder konfektioniert werden.

In einer Ausführungsform werden die Copolymerisate zur Herstellung von Klebstoffzusammensetzungen verwendet. Das Lösungs- bzw. Dispergiermittel der Klebstoffzusammensetzung kann sowohl nur aus Wasser, als auch aus Mischungen aus Wasser und damit mischbaren Flüssigkeiten wie Methanol oder Ethanol bestehen. Vorzugsweise wird nur Wasser verwendet. Der pH-Wert der Polymerdispersion bzw. der Klebstoffzusammensetzung wird vorzugsweise auf pH größer 4,5, insbesondere auf einen pH-Wert zwischen 5 und 9,5 eingestellt. Die Klebstoffzusammensetzungen können allein aus dem Lösungsmittel und dem Polymer bestehen. Die Klebstoffzusammensetzung kann jedoch auch noch weitere Zusatzstoffe enthalten, z.B. Füllstoffe, Farbstoffe, Verlaufsmittel, Verdicker (vorzugsweise Assoziativverdicker), Entschäumer, Pigmente, Netzmittel oder Tackifier (klebrigmachende Harze). Für eine bessere Benetzung von Oberflächen können die Klebstoffe Benetzungshilfsmittel, z. B. Fettalkoholethoxylate, Alkylphenolethoxylate, Nonylphenolethoxylate, Polyoxyethylene, Polyoxypropylene oder Natriumdodecylsulfonate enthalten. Die Menge an Zusatzstoffen beträgt im allgemeinen 0,05 bis 5 Gew.-Teile, insbesondere 0,1 bis 3 Gew.-Teile auf 100 Gew.-Teile Polymer (fest).

Die Klebstoffzusammensetzung ist vorzugsweise ein Haftklebstoff, d.h. sie hat insbesondere nach UV-Vernetzung haftklebende Eigenschaften. Ein Haftklebstoff ist ein viskoelastischer Klebstoff, dessen abgebundener Film bei Raumtemperatur (20°C) in trockenem Zustand permanent klebrig und klebfähig bleibt. Die Klebung auf Substraten erfolgt sofort durch leichten Anpressdruck. Die Klebstoffzusammensetzung kann zur Herstellung von selbstklebenden Artikeln verwendet werden. Die Artikel sind zumindest teilweise mit dem Haftklebstoff beschichtet. In einer Ausführungsform sind die selbstklebenden Artikel nach der Verklebung wiederabziehbar. Bei den selbstklebenden Artikeln kann es sich z.B. um Folien, Bänder oder Etiketten handeln. Selbstklebebänder lassen eine Vielzahl von Einsatzfeldern zu, so z.B. als ein- und doppelseitige Klebebänder, als trägerlose Systeme oder als Pflaster. Geeignete Trägermaterialien sind z.B. Papier, Kunststofffolien, Metallfolien oder textile Träger. Bei den selbstklebenden Bändern kann es sich um einseitig oder beidseitig beschichtete Bänder aus den obigen Substanzen handeln. Bei den selbstklebenden Etiketten kann es sich um Etiketten aus Papier oder einer thermoplastischen Folie handeln. Als thermoplastische Folie kommen z.B. Folien aus Polyolefinen (z.B. Polyethylen, Polypropylen), Polyolefincopolymeren, Folien aus Polyestern (z.B. Polyethylenterephtalat) oder Polyacetat in Betracht. Die Oberflächen der thermoplastischen Polymerfolien sind vorzugweise coronabehandelt. Die Etiketten sind einseitig mit Klebstoff beschichtet. Bevorzugte Substrate für die selbstklebenden Artikel sind Papier und Polymerfolien. Bevorzugte selbstklebende Artikel sind Klebebänder. Die Artikel sind auf mindestens einer Oberfläche zumindest teilweise mit einer Klebstoffzusammensetzung beschichtet. Der Klebstoff kann nach üblichen Methoden wie Rakeln oder Streichen auf die Artikel aufgetragen werden. Die Auftragsmenge beträgt bevorzugt 1 bis 120 g, besonders bevorzugt 30 bis 80 g Feststoff pro $m^2$. Nach dem Auftragen folgen im Allgemeinen ein Trocknungsschritt zur Entfernung des Wasser bzw. der Lösungsmittel sowie eine Vernetzung durch Bestrahlung mit UV-Licht. Bei den Substraten, auf welche die selbstklebenden Artikel vorteilhaft aufgebracht werden können, kann es sich z.B. um Metall, Holz, Glas, Papier oder Kunststoff handeln. Die selbstklebenden Artikel eignen sich insbesondere zum Verkleben auf Verpackungsoberflächen, Kartons, Kunststoffverpackungen, Bücher, Fenster, Kraftfahrzeugkarosserien, Karosserieteilen oder Kabeln.

Die Klebstoffzusammensetzung kann vorteilhaft in der Kabelsatzindustrie zur Herstellung von Selbstklebebändern eingesetzt werden. Je nach Anforderung und Einsatzbereich werden Artikel mit Gewebe, Vlies oder Folien aus unterschiedlichen Materialien eingesetzt. Diese Art Klebebänder kann in allen Bereichen der Automobile eingebaut werden, beispielsweise im Motorraum oder im Innenraum. Beim Kabelbandagieren schützen diese Klebebänder auch vor unerwünschten Wechselwirkungen mit der PVC Aderisolierung. Als Träger für Kabelwickelbänder werden vorzugsweise Gewebe, Vliese Folien, Papier, Filze, Schaumstoffe oder Coextrudate eingesetzt.

Die Klebstoffzusammensetzung kann auch vorteilhaft zur Herstellung von Selbstklebebändern für Medizinprodukte wie z.B. Pflaster und Bandagen eingesetzt werden. Als Trägermaterial für Medizinprodukte eignen sich z.B. Folien, z.B. aus Polypropylen, Polyethylen oder Polyester, Gewebe, z.B. aus Baumwolle, Viskose, Viskoseacetat oder Zellwolle, aber auch Vliese, z.B. aus Viskose oder Polyester sowie anderen Mischungen.

Zur Herstellung von textilen Klebebändern kann die Klebstoffzusammensetzung auf textile Trägermaterialien aufgebracht werden, z.B. Zellwoll-Gewebe, Zellwoll-Gewebe mit Acrylatbeschichtung, Polyester-Gewebe, Polyester-Nähvliese, wasserstrahlverfestigte Polyester-Vliese, Spinnvlies mit einseitiger Kalandrierung, Vliese mit Trennmittelbeschichtung, Nadelvliese mit einer durch Anschmelzen verfestigten Rückseitenoberfläche oder thermisch verfestigte Vliese. Geeignet zur Herstellung auf sich selbst wickelbarer Klebebänder sind z.B. aus einem thermisch verfestigten Vlies bestehende bandförmige Träger welche auf einer Seite mit einer Klebstoffzusammensetzung versehen sind, wobei das Vlies ein Flächengewicht von 10 - 50 $g/m^2$, eine Banddicke von 0,15 - 0,40 mm, eine Reißfestigkeit von 10 - 35 N/cm und eine Reißdehnung von 40-75% aufweisen kann und die Klebebeschichtung aus einem durch ultraviolette Strahlung vemetzbaren Acrylatklebstoff besteht. Es ist aber auch der Einsatz von Vliesen mit höherem Flächengewicht möglich, z.B. ein Klebeband zum Bandagieren von Kabelbäumen mit einem beispielsweise aus Polypropylen bestehenden, thermisch verfestigten Spinnvlies als Träger mit einem Vliesgewicht von 60 bis 100 $g/m^2$, einer Vliesdicke von z.B. 400 bis 600 Mikrometer, einer Fadenfeinheit von z.B. 2 dtex bis 7 dtex, einer Reißkraft von z.B. 200 N/(5 cm) bis 270 N/(5 cm) und einer Reißdehnung von z.B. 55% bis 85%. Die als Träger verwendeten Vliese können beispielsweise aus Polypropylen oder Polyesterfasern bestehen. Beschichtet wird vorzugsweise die glattere Seite, so dass das Band die bei einem Einsatz als Kabelwickelband in der Automobilindustrie gewünschte Geräuschdämpfiungseigenschaft besitzt..

Aufgrund des geringen VOC-Gehalts bzw. der Foggingfreiheit können die Copolymerisate vorteilhaft als Beschichtungen oder Klebstoffe in gegenüber VOC's sensiblen Bereichen angewendet werden, z.B. als Pflaster in der Medizin, im Innenraum von Automobilen, z.B. als Kabelwickelbänder oder als doppelseitige Klebebänder.

Die Lösungen strahlungsempfindlicher Verbindungen können auch vorteilhaft bei der UV-Härtung von Beschichtungsmaterialien, z.B. von dünnen Schichten strahlungshärtbarer Lackbeschichtungen eingesetzt werden. Die Beschichtung kann auf allen hierfür üblichen Trägern erfolgen, z.B. auf Papier, Holz, textilen Trägern, Kunststoff oder Metall. Ein weiteres Anwendungsgebiet ist die Trocknung bzw. Härtung von Druckfarben und Siebdruckmaterialien, insbesondere bei der Oberflächenbeschichtung oder der Oberflächengestaltung von Dosen, Tuben und metallenen Verschlusskappen. Aufgrund des stark verringerten Gehalts an leichtflüchtigen Anteilen und der Freiheit von aromatischen Kohlenwasserstoffen nach erfolgter Härtung sind solche Anwendungen besonders vorteilhaft, bei denen eine Diffusion oder Migration von Stoffen in die an die Beschichtung angrenzenden Trägermaterialien minimiert oder ausgeschlossen werden soll, z.B. bei beschichteten Verpackungsmaterialien, welche mit Lebensmitteln in Berührung kommen.

Ein Anwendungsgebiet ist die Herstellung von strahlungshärtbaren Beschichtungszusammensetzungen. Strahlungshärtbare Zusammensetzungen haben in der Technik eine breite Anwendung gefunden, insbesondere als hochwertige Beschichtungsmaterialien für Oberflächen. Unter strahlungshärtbaren Zusammensetzungen versteht man Zubereitungen, die ethylenisch ungesättigte Polymere oder Prepolymere enthalten und die, gegebenenfalls nach einem physikalischen Trocknungsschritt, durch Einwirkung energiereicher Strahlung, z.B. durch Bestrahlung mit UV-Licht ausgehärtet werden.

Geeignete härtbare Bestandteile von strahlungshärtbaren Beschichtungszusammensetzungen sind z.B. ethylenisch ungesättigte Urethane (A), monoethylenisch ungesättigte Reaktivverdünner (B) oder allgemein multifunktionelle polymerisationsfähige Verbindungen (C) mit mehr als einer, bevorzugt mindestens zwei radikalisch polymerisationsfähigen Gruppen. Diese Stoffe können allein oder in Kombination eingesetzt werden. Geeignete Bestandteile (A), (B) und (C) und deren Kombinationen sind z.B. in der WO 2008/155352 beschrieben. Ethylenisch ungesättigte Urethane (A) sind beispielsweise aliphatische oder cycloaliphatische Urethan(meth)acrylate mit zwei oder mehr ethylenisch ungesättigten Doppelbindungen pro Molekül, deren Polymere, Oligomere oder Prepolymere. Geeignete monoethylenisch ungesättigte Reaktivverdünner (B) sind beispielsweise Verbindungen die wenigstens eine cycloaliphatische oder mindestens eine heterocyclische Gruppe enthalten, z.B. Ester der Acrylsäure oder der Methacrylsäure mit Cycloalkanolen oder Bicycloalkanolen, wobei das Cycloalkanol oder Bicycloalkanol von 3 bis 20 C-Atomen, bevorzugt 5 bis 10 C-Atome aufweist und optional mit $C_1$- bis $C_4$-Alkyl substituiert sein kann. Beispiele für Cycloalkanol und Bicycloalkanol sind Cyclopentanol, Cyclohexanol, Cyclooctanol, Cyclododecanol, 4-Methyl cyclohexanol, 4-iso Propyl cyclohexanol, 4-tert. Butyl cyclohexanol (bevorzugt cis konfiguriert), Dihydrodicyclopentadienylalkohol, Isoborneol und Norbornylalkohol. Bevorzugt ist Isoborneol, Cyclohexanol und 4-tert. Butyl cyclohexanol. Reaktivverdünner mit heterocyclischer Gruppe sind z.B. monofunktionelle Ester $\alpha,\beta$-ethylenisch ungesättigter Carbonsäuren (vorzugsweise Acrylsäure oder Methacrylsäure) mit einem monofunktionellen Alkanolen, welche wenigstens einen gesättigten 5- oder 6-gliedrigen Heterocyclus mit einem oder zwei Sauerstoffatomen im Ring als Strukturelement aufweisen. Vorzugsweise leitet sich der 5- oder 6-gliedrige, gesättigte Heterocyclus von Tetrahydrofuran, Tetrahydropyran, 1,3-Dioxolan, 1,3- oder 1,4-Dioxan ab. Besonders bevorzugt sind Trimethylolpropanmonoformalacrylat, Glycerinmonoformalacrylat, 4-Tetrahydropyranylacrylat, Isobornylacrylat, 2-Tetrahydropyranylmethylacrylat, Tetra-hydrofurfurylacrylat und Mischungen davon. Geeignete multifunktionelle Verbindungen (C) sind beispielsweise multifunktionelle (Meth)acrylate, die mehr als 1, bevorzugt 2 bis 10,

besonders bevorzugt 2 bis 6 oder 2 bis 4 oder 2 bis 3 (Meth)acrylatgruppen, bevorzugt Acrylatgruppen tragen, z.B. Ester der (Meth)acrylsäure mit entsprechend mindestens zweiwertigen Polyalkoholen. Polyalkohole sind beispielsweise mindestens zweiwertige Polyole, Polyether- oder Polyesterole oder Polyacrylatpolyole mit einer mittleren OH-Funktionalität von mindestens 2, bevorzugt 3 bis 10. Beispiele für multifunktionelle, polymerisationsfähige Verbindungen (C) sind Ethylenglykoldiacrylat, 1,2-Propandioldiacrylat, 1,3-Propandioldiacrylat, 1,4-Butandioldiacrylat, 1,3-Butandioldiacrylat, 1,5-Pentandioldiacrylat, 1,6-Hexandioldiacrylat, 1,8-Octandioldiacrylat, Neopentylglykoldiacrylat, 1,1-, 1,2-, 1,3- und 1,4-Cyclohexandimethanoldiacrylat, 1,2-, 1,3- oder 1,4-Cyclohexandioldiacrylat, Trimethylolpropantriacrylat, Ditrimethylolpropanpenta- oder -hexaacrylat, Pentaerythrittri- oder -tetraacrylat, Glycerindioder -triacrylat, sowie Di- und Polyacrylate von Zuckeralkoholen, wie beispielsweise Sorbit, Mannit, Diglycerol, Threit, Erythrit, Adonit (Ribit), Arabit (Lyxit), Xylit, Dulcit (Galactit), Maltit oder Isomalt, oder von Polyesterpolyclen, Polyetherolen, Poly-1,3-Propandiol mit einer Molmasse zwischen 134 und 1178, Polyethylenglykol mit einer Molmasse zwischen 106 und 898, sowie Epoxy(meth)acrylate, Urethan(meth)acrylate oder Polycarbonat(meth)acrylate. Bevorzugte multifunktionelle, polymerisationsfähige Verbindungen (C) sind Ethylenglykoldiacrylat, 1,2-Propandioldiacrylat, 1,3-Propandioldiacrylat, 1,4-Butandioldiacrylat, 1,6-Hexandioldiacrylat, Trimethylolpropantriacrylat, Pentaerythrittetraacrylat, Polyestepolyolacrylate, Polyetherolacrylate und (Meth)Acrylate von ein- bis zwanzigfach und besonders bevorzugt drei- bis zehnfach ethoxyliertem, propoxyliertem oder gemischt ethoxyliertem und propoxyliertem und insbesondere ausschließlich ethoxyliertem Neopentylglykol, Trimethylolpropan, Trimethylolethan oder Pentaerythrit, z.B Triacrylat von ein- bis zwanzigfach alkoxyliertem, besonders bevorzugt ethoxyliertem Trimethylolpropan.

Die strahlungshärtbare Beschichtungszusammensetzung kann den erfindungsgemäß hergestellten Fotoinitiator in einer Menge von beispielsweise 0,1 bis 10 Gew.%, bezogen auf die Gesamtmenge der Verbindungen (A), (B) und (C) enthalten. Zusätzlich können bezogen jeweils auf die Summe der Verbindungen (A), (B) und (C) 0 bis 10, vorzugsweise 0,1 bis 10 Gew.% mindestens eines UV Stabilisators (E), 0 bis 5, vorzugsweise 0,1 bis 5 Gew.% an geeigneten Radikalfängern (F) und 0 bis 10, vorzugsweise 0,1 bis 10 Gew.% weiterer lacktypischer Additive (G) enthalten sein. Geeignete Stabilisatoren (E) umfassen z.B. typische UV-Absorber wie Oxanilide, Triazine und Benzotriazol (letztere erhältlich als Tinuvin® -Marken der Ciba-Spezialitäten-chemie) und Benzophenone. Geeignete Radikalfänger (F) sind z.B. sterisch gehinderte Amine wie 2,2,6,6-Tetramethylpiperidin, 2,6-Di-tert.-butylpi-peridin oder deren Derivate, z. B. Bis-(2,2,6,6-tetra-methyl-4-piperidyl)sebacinat. Lacktypische Additive (G) können beispielsweise Antioxidantien, Aktivatoren (Beschleuniger), Füllmittel, Pigmente, Farbstoffe, antistatische Agenzien, Flammschutzmittel, Verdicker, thixotrope Agenzien, oberflächenaktive Agenzien, Viskositätsmodifikatoren, Plastifizierer oder Chelatbildner sein. Verdicker sind z.B. radikalisch (co)polymerisierte (Co)Polymerisate, übliche organische und anorganische Verdicker wie Hydroxymethylcellulose oder Bentonit. Chelatbildner können z.B. Ethylendiaminessigsäure und deren Salze sowie β-Diketone sein. Füllstoffe umfassen beispielsweise Silikate, z. B. durch Hydrolyse von Siliciumtetrachlorid erhältliche Silikate wie Aerosil® der Fa. Degussa, Kieselerde, Talkum, Aluminiumsilikate, Magnesiumsilikate, Calciumcarbonate etc.

Die Beschichtungsmassen eignen sich zum Beschichten von Substraten wie Holz, Papier, Textil, Leder, Vlies, Kunststoffoberflächen, Glas, Keramik, mineralischen Baustoffen, wie Zementformsteine und Faserzementplatten, oder Metallen oder beschichteten Metallen, bevorzugt von Kunststoffen oder Metallen, insbesondere in Form von Folien, besonders bevorzugt Metallen. Die Beschichtungsmittel können insbesondere in Grundierungen, Füllern, pigmentierten Decklacken und Klarlacken im Bereich Autoreparatur- oder Großfahrzeuglackierung und Flugzeugen eingesetzt werden. Insbesondere können die Beschichtungsmassen als oder in Automobilklar- und -decklacke(n) eingesetzt werden. Weitere bevorzugte Einsatzgebiete sind Can-Coating und Coil-Coating.

Beispiele

Beispiel 1 (erfindungsgemäß): Polymerisierbarer Fotoinitiator

In einen mit Magerluft (Sauerstoffanteil 6%) teilinertisierten Reaktor werden 700 kg 4-Hydroxybenzophenon eingefüllt. Unter permanentem Durchstrom von Magerluft werden 2250 kg Methylethylketon (MEK; frisch oder aus einem früheren Ansatz zurück gewonnen) und 375 kg Triethylamin zugepumpt. Der Ansatz wird mit 175 g 2,2,6,6-Tetramethylpiperidinyloxyl (TEMPO) stabilisiert. Danach werden bei einer Innentemperatur von 40°C 737 kg Acryloxybutylchlorformiat zudosiert und 20 h nachgerührt. Danach werden 1030 kg 5%ige Salzsäure zudosiert. Die untere, wässrige Phase wird abgetrennt und die organische Wertproduktphase mit 150 g TEMPO stabilisiert. Zur Abtrennung von restlichem Triethylamin und Wasseranteilen wird eine Teilmenge des vorhandenen Lösungsmittels abdestilliert. Danach wird mit 2,5 kg Hydrochinonmonomethylether-Lösung in MEK stabilisiert und auf die gewünschte Konzentration mit Methylethylketon verdünnt. Der Reaktorinhalt wird auf -10°C abgekühlt und über einen Filter abgefüllt. Es wird eine Lösung des polymerisierbaren Fotoinitiators in Methylethylketon mit einem Gehalt von 35 Gew.-% und in einer Ausbeute von ca. 93 % (bezogen auf 4-Hydroxybenzophenon) erhalten.

Herstellung der Copolymere und des Schmelzhaftklebers:

Es wird eine Copolymerlösung hergestellt durch radikalische Polymerisation folgender Bestandteile in Methylethylketon:

| | |
|---|---|
| Vorlage: | 15,5 kg Methylethylketon |
| Zulauf 1: | 79,5 kg n-Butylacrylat |
| | 4,2 kg Acrylsäure |
| | 0,6 kg Fotoinitiator gem. Beispiel 1 (gelöst in Methylethylketon, ca. 35%ig) |
| Zulauf 2: | 0,1 kg Radikalstarter |

Man polymerisiert bei einer Temperatur von 81°C bei einem Zulauf der Komponenten innerhalb von 4 Stunden. K-Wert des Copolymers: 48-52.

Durch Verdampfen des Lösungsmittels wird aus der Copolymerlösung ein Schmelzhaftklebstoff hergestellt.

Beispiel 2 (nicht erfindungsgemäß, Stand der Technik)

Es werden ein polymerisierbarer Fotoinitiator, eine Copolymerlösung und ein Schmelzhaftklebstoff wie in Beispiel 1 hergestellt, mit dem Unterschied, dass die Herstellung des Fotoinitiators in o-Xylol anstelle von Methylethylketon erfolgt und zur Herstellung der Copolymerlösung eine Lösung des Fotoinitiators in o-Xylol (ca. 35%ig) eingesetzt wird.

Eigenschaften:

Die Eigenschaften der nach Beispiel 1 und Beispiel 2 hergestellten Schmelzhaftklebstoffe sind in Tabelle 1 zusammengefasst.

Tabelle 1

| | Beispiel 1 | Beispiel 2 |
|---|---|---|
| VOC-Gehalt | 160 ppm | 240 ppm |
| Gehalt flüchtiger aromatischer Kohlenwasserstoffe | 0 ppm | 70 ppm |
| Gelbwert [1] | 50 | 80 |
| [1] Platin-Cobalt-Farbzahl (HAZEN-Farbzahl) nach DIN EN ISO 6271-1 | | |

Beispiel 3: Kabelwickelband

Die Herstellung eines Kabelwickelbandes kann beispielsweise wie folgt erfolgen. Auf einen Vliesträger (Maliwatt, Flächengewicht 80g/m$^2$, Feinheit 22, zum Beispiel von der Fa. Cottano) wird mit einer Rollstabdüse eine UV-vernetzbare Acrylat-Hot-Melt-Klebemasse gemäß Beispiel 1 mit einer Geschwindigkeit von 50 m/min aufgetragen. Die Beschichtung kann dabei auf unterschiedliche Weisen erfolgen. Beispielsweise können in einer Variante im Direktstrich 80 g/m$^2$ Klebemasse mit einer Temperatur von 90 bis 110 °C auf das Vlies aufgetragen werden, wobei die Streichunterwelle temperiert wird. In einer anderen Variante können 50 g/m$^2$ Klebemasse auf ein Gurtband beschichtet und in einer temperierbaren Kaschierstation mit 80 °C und einem Druck von 8 bar auf den Vliesträger transferiert werden. Diese Variante lässt eine besonders komfortable Steuerung der Verankerung der Klebmasse auf dem Trägermaterial unter Vermeidung eines unzulässigen Massedurchschlags zu. Die beschichteten Vliesträger werden im weiteren Bahnverlauf der Anlage mit UV-Strahlern vernetzt (z.B. mit 6 Mitteldruck Hg-Lampen à 120 W/cm). Der Vernetzungsgrad kann über die UV-Dosis variabel eingestellt werden, so dass die klebtechnischen Eigenschaften (u.a. Klebkraft, Abrollkraft) individuell eingestellt werden können. Die beschichteten und bestrahlten Vliesträger können an einem Stangenwickler zu den gewünschten Längen auf Stangen aufgewickelt und an einem Abstechautomaten zu den gewünschten Breiten konfektioniert werden.

Das Klebeband zeichnet sich durch einen besonders geringen Gehalt an leichtflüchtigen organischen Stoffen, insbesondere durch eine Freiheit an flüchtigen aromatischen Kohlenwasserstoffen aus.

Beispiel 4: Pflaster

Die Herstellung eines Pflasters kann beispielsweise wie folgt erfolgen. Auf einen Pflasterfolienträger aus Polyolefin mit einer flächenspezifischen Masse von 56 g/m$^2$ wird mit einer Rollstabdüse bei einer Geschwindigkeit von 80 m/min eine UV-vernetzbare. Acrylat-Hot-Melt-Klebemasse gemäß Beispiel 1 in einer Menge von 38 g/m$^2$ und mit einer

Temperatur von 145 °C aufgetragen. Eine ausreichende Verankerung auf dem Träger kann durch eine Temperierung der Streichunterwelle erreicht werden. Das beschichtete Material wird durch Bestrahlung mit ultraviolettem Licht, beispielsweise von vier Mitteldruck Hg-Strahlern à 120 W/cm vernetzt. Über die UV-Dosis kann der Vernetzungsgrad variabel eingestellt werden, so dass die entsprechenden klebtechnischen Eigenschaften (u.a. Klebkraft, Abrollkraft) individuell eingestellt werden kann. Die beschichtete Folie wird mit einem silikonisierten Papier kaschiert, zu einem Ballen gewickelt und zu Klebebandrollen weiterverarbeitet.

Das Pflaster zeichnet sich durch einen besonders geringen Gehalt an leichtflüchtigen organischen Stoffen, insbesondere durch eine Freiheit an flüchtigen aromatischen Kohlenwasserstoffen aus.

Beispiel 5: Auf sich selbst wickelbares Kabelwickelband

Die Herstellung eines auf sich selbst wickelbaren Kabelwickelbandes kann beispielsweise wie folgt erfolgen. Auf ein Polypropylen-Spinnvlies mit einem Flächengewicht von 40 $g/m^2$ und einer Materialdicke von 0,3 mm werden 80 $g/m^2$ einer UV-vernetzbaren Acrylat-Hot-Melt-Klebemasse gemäß Beispiel 1 aufgebracht. Alternativ kann auch ein Polyesterspinnvlies verwendet werden. Der fertig beschichtete Träger kann mit geringer Zugspannung auf sich selbst gewickelt werden. Durch Aufbringen einer Prägung kann die geräuschdämmende Wirkung für einen Einsatz des Klebebands zum Umwickeln von Kabelsätzen weiter verbessert werden. Der Vernetzungsgrad des Klebstoffs und die Wickelparameter bei Beschichtung und Konfektionierung können so gewählt werden, dass ein Verblocken des Bandes nicht auftritt.

Das Klebeband zeichnet sich durch einen besonders geringen Gehalt an leichtflüchtigen organischen Stoffen, insbesondere durch eine Freiheit an flüchtigen aromatischen Kohlenwasserstoffen aus.

Beispiel 6: Auf sich selbst wickelbares Allzweck-Klebeband

Die Herstellung eines auf sich selbst wickelbaren Allzweck-Klebebandes kann beispielsweise wie folgt erfolgen. Auf ein Polypropylen-Spinnvlies mit einem Flächengewicht von 50 $g/m^2$ und einer Materialdicke von 0,5 mm werden 90 $g/m^2$ einer erUV-vernetzbaren Acrylat-Hot-Melt-Klebemasse gemäß Beispiel 1 aufgebracht. Alternativ kann auch ein Polyesterspinnvlies verwendet werden. Der fertig beschichtete Träger wird mit geringer Zugspannung auf sich selbst gewickelt.

Das Klebeband zeichnet sich durch einen besonders geringen Gehalt an leichtflüchtigen organischen Stoffen, insbesondere durch eine Freiheit an flüchtigen aromatischen Kohlenwasserstoffen aus.

## Patentansprüche

1. Verfahren zur Herstellung von Lösungen von strahlungsempfindlichen, radikalisch polymerisierbaren organischen Verbindungen, wobei

    a) eine erste Ausgangsverbindung, welche eine Säurehalogenidgruppe aufweist, und
    b) eine zweite Ausgangsverbindung, welche eine alkoholische Hydroxygruppe aufweist,

    miteinander in einem Lösungsmittel oder in einem Lösungsmittelgemisch verestert werden; und
    wobei es sich bei dem Lösungsmittel um ein oder mehrere Ketone mit einem Siedepunkt von unter 150°C bei Normaldruck (1 bar) handelt oder das Lösungsmittelgemisch zu mindestens 50 Gew.%, bezogen auf die Lösungsmittelmenge, aus diesen Ketonen besteht; und
    eine der beiden Ausgangsverbindungen mindestens eine strahlungsempfindliche Gruppe aufweist und die andere der beiden Ausgangsverbindungen mindestens eine ethylenisch ungesättigte, radikalisch polymerisierbare Gruppe aufweist, wobei es sich bei der strahlungsempfindlichen, radikalisch polymerisierbaren organischen Verbindung um einen polymerisierbaren Fotoinitiator handelt, welcher durch radikalische Copolymerisation in die Polymerkette von Copolymeren eingebaut werden kann,
    wobei die Veresterung in Gegenwart von mindestens einem tertiären organischen Amin erfolgt, und
    wobei im Anschluss an die Reaktion Wasser in einer Menge zugegeben wird, dass sich eine gesättigte Lösung des organischen Ammoniumhalogenids in Wasser bildet und diese gesättigte wässrige Lösung von der organischen Phase abgetrennt wird, wobei die Wassermenge so bemessen ist, dass sie die zur Bildung einer gesättigten Lösung benötigte Menge nicht um mehr als 10 Gew.% der theoretisch benötigten Menge überschreitet.

2. Verfahren nach Anspruch 1, **dadurch gekennzeichnet, dass** die erste Ausgangsverbindung eine Chlorformiatgruppe und eine Acrylat- oder Methacrylatgruppe aufweist und die zweite Ausgangsverbindung eine alkoholische Hydroxygruppe und eine Phenongruppe aufweist.

3. Verfahren nach einem der vorhergehenden Ansprüche, **dadurch gekennzeichnet, dass** die Veresterung in Gegenwart von mindestens einem tertiären organischen Amin erfolgt und die Menge an eingesetztem Amin stöchiometrisch ist.

4. Verfahren nach dem vorhergehenden Anspruch, **dadurch gekennzeichnet, dass** nach Abtrennung der gesättigten wässrigen Lösung in der organischen Phase gelöst verbliebene Restanteile an Ammoniumsalzen durch Temperaturabsenkung ausgefällt und abgetrennt werden.

5. Verfahren nach einem der vorhergehenden Ansprüche, **dadurch gekennzeichnet, dass** zumindest ein Teil der als Lösungsmittel eingesetzten Ketone recyclierte, wasserhaltige Ketone sind, welche aus einem vorhergehenden Herstellungsverfahren zurückgewonnen wurden.

6. Verfahren nach einem der vorhergehenden Ansprüche, **dadurch gekennzeichnet, dass** überschüssiges Amin durch Neutralisation mit Säure und/oder destillativ entfernt wird.

7. Verfahren nach Anspruch 1, **dadurch gekennzeichnet, dass** die strahlungsempfindliche, radikalisch polymerisierbare organische Verbindung die allgemeine Struktur A-X-B aufweist, wobei
A ein einwertiger organischer Rest ist, welcher eine Phenongruppe aufweist,
X eine Estergruppe ist, ausgewählt aus -O-C(=O)-, -(C=O)-O und -O-(C=O)-O-, und B ein einwertiger organischer Rest ist, welcher eine ethylenisch ungesättigte, radikalisch polymerisierbare Gruppe enthält.

8. Verfahren nach Anspruch 7, **dadurch gekennzeichnet, dass** die strahlungsempfindliche, radikalisch polymerisierbare organische Verbindung die allgemeine Formel

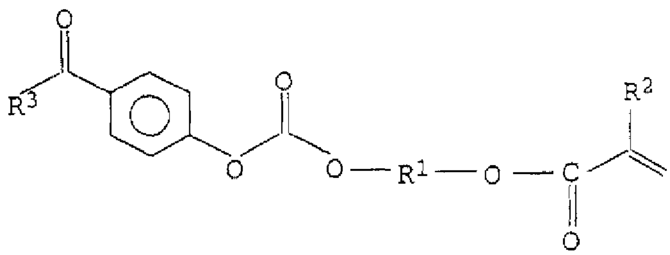

aufweist, worin R1 für einen divalenten organischen Rest mit bis zu 30 C-Atomen, R2 für ein H-Atom oder eine Methylgruppe und R3 für eine substituierte oder unsubstituierte Phenylgruppe oder für eine C1-C4-Alkylgruppe steht.

9. Verfahren nach einem der Ansprüche 7 bis 8, **dadurch gekennzeichnet, dass** es sich bei den Lösungsmittelketonen um Dialkylketone mit einem Siedepunkt im Bereich von 50 bis unter 130 °C bei 1 bar handelt und der Gehalt an radikalisch polymerisierbaren organischen Verbindungen von 5 bis 85, vorzugsweise 20 bis 60 Gew.-% beträgt.

## Claims

1. A process for preparing a solution of radiation-sensitive, free-radically polymerizable organic compounds, where

   a) a first starting compound, which has an acid halide group, and
   b) a second starting compound, which has an alcoholic hydroxyl group,

are esterified with one another in a solvent or solvent mixture; and
where the solvent comprises one or more ketones having a boiling point of below 150°C under atmospheric pressure (1 bar) or the solvent mixture is composed to an extent of at least 50% by weight, based on the amount of solvent, of said ketones; and one of the two starting compounds has at least one radiation-sensitive group and the other of the two starting compounds has at least one ethylenically unsaturated, free-radically polymerizable group, where the radiation-sensitive, free-radically polymerizable organic compound comprises a polymerizable photo-initiator which can be incorporated into the polymer chain of copolymers by means of free-radical copolymerization,

where the esterification takes place in the presence of at least one tertiary organic amine, and
where after the reaction water is added in an amount such as to form a saturated solution of the organic ammonium halide in water, and this saturated aqueous solution is separated from the organic phase, the amount of water being calculated such that it does not exceed the amount required to form a saturated solution by more than 10% by weight of the amount required theoretically.

2. The process according to claim 1, wherein the first starting compound has a chloroformate group and an acrylate or methacrylate group and the second starting compound has an alcoholic hydroxyl group and a phenone group.

3. The process according to either of the preceding claims, wherein the esterification takes place in the presence of at least one tertiary organic amine and the amount of amine used is stoichiometric.

4. The process according to the preceding claim, wherein, after the saturated aqueous solution has been separated, residual fractions of ammonium salts that have remained in solution in the organic phase are precipitated by temperature reduction and separated.

5. The process according to any of the preceding claims, wherein at least part of the ketone solvents used are recycled hydrous ketones recovered from a prior preparation process.

6. The process according to any of the preceding claims, wherein excess amine is removed by neutralization with acid and/or by distillation.

7. The process according to claim 1, wherein the radiation-sensitive, free-radically polymerizable organic compound has the general structure
A-X-B, where
A is a monovalent organic radical which has a phenone group,
X is an ester group selected from -O-C(=O)-, -(C=O)-O-, and -O-(C=O)-O-,
and B is a monovalent organic radical which comprises an ethylenically unsaturated, free-radically polymerizable group.

8. The process according to claim 7, wherein the radiation-sensitive, free-radically polymerizable organic compound has the general formula

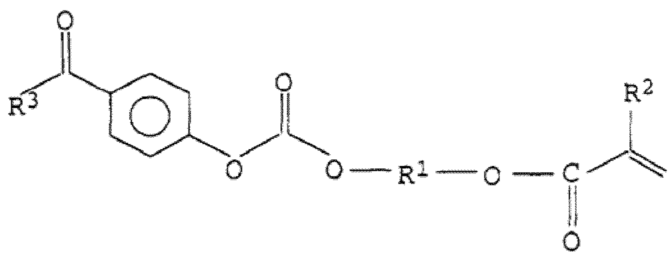

in which R1 is a divalent organic radical having up to 30 C atoms, R2 is an H atom or a methyl group, and R3 is a substituted or unsubstituted phenyl group or is a C1-C4 alkyl group.

9. The process according to either of claims 7 to 8, wherein the ketone solvents are dialkyl ketones having a boiling point in the range from 50 to below 130 °C at 1 bar and the amount of free-radically polymerizable organic compounds is from 5% to 85%, preferably 20% to 60%, by weight.

**Revendications**

1. Procédé pour la préparation de solutions de composés organiques, polymérisables par voie radicalaire, sensibles à un rayonnement,

a) un premier composé de départ, qui présente un groupe halogénure d'acide, et
b) un deuxième composé de départ, qui présente un groupe hydroxy alcoolique,

étant estérifiés l'un avec l'autre dans un solvant ou un mélange de solvants ; et le solvant étant une ou plusieurs cétones présentant un point d'ébullition inférieur à 150°C à pression normale (1 bar) ou le mélange de solvants étant constitué à raison d'au moins 50% en poids, par rapport à la quantité de solvant, de ces cétones ; et un des deux composés de départ présentant au moins un groupe sensible à un rayonnement et l'autre des deux composés de départ présentant au moins un groupe polymérisable par voie radicalaire, éthyléniquement insaturé, le composé organique polymérisable par voie radicalaire, sensible à un rayonnement étant un photo-initiateur polymérisable, qui peut être incorporé par copolymérisation radicalaire dans la chaîne polymère de copolymères, l'estérification ayant lieu en présence d'au moins une amine organique tertiaire et de l'eau étant ajoutée après la réaction en une quantité telle qu'il se forme une solution saturée de l'halogénure d'ammonium organique dans de l'eau et cette solution aqueuse saturée étant séparée de la phase organique, la quantité d'eau étant dimensionnée de manière telle qu'elle ne dépasse pas la quantité nécessaire pour la formation d'une solution saturée de plus de 10% en poids de la quantité théoriquement nécessaire.

2. Procédé selon la revendication 1, **caractérisé en ce que** le premier composé de départ présente un groupe chloroformiate et un groupe acrylate ou méthacrylate et le deuxième composé de départ présente un groupe hydroxy alcoolique et un groupe phénone.

3. Procédé selon l'une quelconque des revendications précédentes, **caractérisé en ce que** l'estérification a lieu en présence d'au moins une amine organique tertiaire et la quantité de l'amine utilisée est stoechiométrique.

4. Procédé selon la revendication précédente, **caractérisé en ce que** des proportions résiduelles de sels d'ammonium qui restent sous forme dissoute dans la phase organique après la séparation de la solution aqueuse saturée sont précipitées par diminution de la température et séparées.

5. Procédé selon l'une quelconque des revendications précédentes, **caractérisé en ce qu'**au moins une partie des cétones utilisées comme solvant sont des cétones recyclées contenant de l'eau qui ont été récupérées d'un procédé de préparation préalable.

6. Procédé selon l'une quelconque des revendications précédentes, **caractérisé en ce que** l'amine en excès est éliminée par neutralisation avec un acide et/ou par distillation.

7. Procédé selon la revendication 1, **caractérisé en ce que** le composé organique polymérisable par voie radicalaire, sensible à un rayonnement présente la structure générale A-X-B,
A étant un radical organique monovalent, qui présente un groupe phénone,
X étant un groupe ester choisi parmi -O-C(=O)-, -(C=O)-O et -O-(C=O)-O-, et
B étant un radical organique monovalent, qui contient un groupe polymérisable par voie radicalaire, éthyléniquement insaturé.

8. Procédé selon la revendication 7, **caractérisé en ce que** le composé organique polymérisable par voie radicalaire, sensible à un rayonnement présente la formule générale

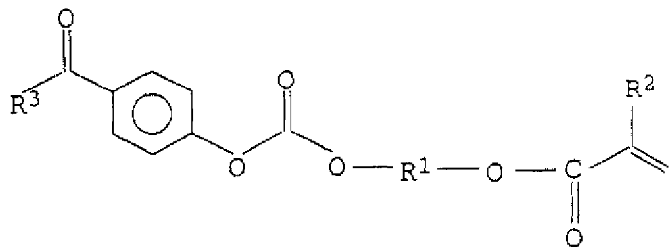

dans laquelle $R^1$ représente un radical organique divalent, comprenant jusqu'à 30 atomes de carbone, $R^2$ représente un atome H ou un groupe méthyle et $R^3$ représente un groupe phényle substitué ou non substitué ou un groupe $C_1$-$C_4$-alkyle.

9. Procédé selon l'une quelconque des revendications 7 à 8, **caractérisé en ce qu'**il s'agit, pour les cétones de solvant, de dialkylcétones présentant un point d'ébullition dans la plage de 50 à moins de 130°C à 1 bar et la teneur en composés organiques polymérisables par voie radicalaire est de 5 à 85, de préférence de 20 à 60% en poids.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 377199 A
- EP 655465 A
- EP 1213306 A
- EP 377191 A
- JP 4038600 B
- WO 2008155352 A


### In der Beschreibung aufgeführte Nicht-Patentliteratur


- **W. MÄCHTLE.** *Makromolekulare Chemie,* 1984, vol. 185, 1025-1039